# EUROPÄISCHE PATENTANMELDUNG

(11) **EP 1 428 551 A1**
(43) Veröffentlichungstag der Anmeldung: **16.06.2004**
(21) Anmeldenummer: 03027023.5
(22) Anmeldetag: 24.11.2003
(51) Int. Cl.: A61N 1/05

(54) **Stimulationselektrode, sowie deren Herstellung und Verwendung**

(30) Priorität: 13.12.2002 DE 10258651
(71) Anmelder: W.C. Heraeus GmbH & Co. KG, 63450 Hanau (DE)
(72) Erfinder: Frericks, Matthias, 63456 Hanau (DE); Specht, Heiko, 63739 Aschaffenburg (DE); Krüger, Frank, Dr., 63486 Bruchköbel (DE); Keitel, Oliver, 63741 Aschaffenburg (DE); Leitold, Christiane, 61200 Wölfersheim (DE); Wachter, Jürgen, Dr., 63322 Rödermark (DE)
(74) Vertreter: Kühn, Hans-Christian

(57) **Zusammenfassung**

Die Erfindung betrifft eine Stimulationselektrode mit einem elektrisch leitenden Elektrodengrundkörper, der teilweise mit einer elektrisch isolierenden Keramikschicht bedeckt ist, wobei die Keramikschicht aus einem Oxid und/oder einem Oxidnitrid mindestens eines der Metalle der Gruppe umfassend Titan, Niobium, Tantal, Zirkonium, Aluminium und Silizium gebildet ist. Die Erfindung betrifft weiterhin die Verwendung einer solchen Stimulationselektrode und unterschiedliche Verfahren zu ihrer Herstellung.

## Beschreibung

Die Erfindung betrifft eine Stimulationselektrode mit einem elektrisch leitenden Elektrodengrundkörper, der teilweise mit einer elektrisch isolierenden Keramikschicht bedeckt ist, wobei die Keramikschicht aus einem Oxid und/oder einem Oxinitrid mindestens eines Metalls der Gruppe umfassend Titan, Niobium, Tantal, Zirkonium, Aluminium und Silizium gebildet ist, sowie die Verwendung einer solchen Stimulationselektrode. Die Erfindung betrifft weiterhin unterschiedliche Verfahren zur Herstellung einer erfindungsgemäßen Stimulationselektrode.

Die EP 1 169 972 A offenbart einen Katheter, der mit einer Stimulationselektrode ausgestattet ist. Im Bereich der Stimulationselektrode ist eine elektrisch isolierende sowie thermisch leitfähige Schicht angeordnet, die unter anderem aus Keramik gebildet sein kann. Die Dicke der elektrisch isolierenden und thermisch leitfähigen Schicht beträgt dabei <10 µm.

Die EP 0 620 024 B1 offenbart eine Stimulationselektrode, die auf der Elektronenspitze eine hochohmige Isolationsschicht aufweist. Besonders bevorzugt ist dabei ein diamantähnlicher Kohlenstoff, welcher extrem biokompatibel ist. Der diamantähnliche Kohlenstoff kann dabei unter anderem mit Hilfe von Laser aufgelegt werden. Weiterhin ist offenbart, den gesamten Elektrodenkopf mit einer diamantähnlichen Kohlenstoffschicht zu belegen und danach die Stimulationsflächen mittels Fotoätzung nach Wunsch freizulegen.

Die US 6,298,272 B1 offenbart eine Herzschrittmacherelektrode, die im Bereich der Elektrodenspitze mit einer isolierenden Beschichtung versehen ist. Die isolierende Beschichtung kann dabei z.B. durch Tauchen, Drucken, Aufsprühen, Aufpinseln und Resisttechniken gebildet werden.

Die WO 98/31419 offenbart eine Herzschrittmacherelektrode, bei der die Elektrodenspitze eine ringförmige, elektrisch isolierende Umhüllung aufweist. Als Material für diese Umhüllung sind dabei Kunststoffe bzw. Elastomere, insbesondere Silikongummi offenbart.
Die DE 35 18 317 A1 offenbart eine Stimulationselektrode, die teilweise mit Isolationsmaterial beschichtet ist. Als Beschichtungsmaterial ist dabei Kunststoff offenbart.

Es ist nun Aufgabe der Erfindung, eine Stimulationselektrode mit sehr guter Biokompatibilität und hoher Langzeitkorrosionsbeständigkeit anzugeben, die gleichzeitig schnell kostengünstig hergestellt werden kann.

Die Aufgabe wird für die Stimulationselektrode dadurch gelöst, dass der Elektrodengrundkörper weiterhin zumindest teilweise mit einer elektrisch leitfähigen Beschichtung aus Titannitrid, Niobiumnitrid, Tantalnitrid, Zirkoniumnitrid, Aluminiumnitrid, Siliziumnitrid, Vanadiumnitrid, Iridiumoxid oder einer Legierung aus Platin und Iridium beschichtet ist, wobei der Iridium-Anteil der Legierung ≥ 21 Gew.-% beträgt und der Platin-Anteil der Legierung ≥ 100 ppm beträgt.
Diese Materialien weisen eine besonders gute Biokompatibilität auf und können mit ausgezeichneter Haftfestigkeit an elektrisch leitenden Elektrodengrundkörpern angeordnet werden. Die nachfolgende Beschreibung geeigneter Herstellungsverfahren für die Keramikschicht zeigt, dass diese Materialien in einfacher und kostengünstiger Weise einsetzbar sind.

Insbesondere hat es sich dabei bewährt, wenn der Elektrodengrundkörper dabei aus Titan, Tantal, Gold, Kohlenstoff, Platin, Iridium, einer Platin-Iridium-Legierung, einer auf Kobalt und/oder Nickel basierenden Legierung oder Edelstahl gebildet ist.

Die Keramikschicht kann dabei auf der elektrisch leitfähigen Beschichtung angeordnet sein. Die Keramikschicht kann aber auch neben der elektrisch leitfähigen Beschichtung auf dem Elektrodengrundkörper angeordnet sein.

Außerdem hat es sich bewährt, den Elektrodengrundkörper zumindest teilweise mit einer elektrisch leitfähigen Beschichtung aus Titannitrid zu beschichten. Dabei kann die Keramikschicht sowohl auf der elektrisch leitfähigen Beschichtung aus Titannitrid angeordnet sein als auch neben der elektrisch leitfähigen Beschichtung aus Titannitrid auf dem Elektrodengrundkörper.

Dabei ist es von Vorteil, wenn die elektrisch leitfähige Beschichtung aus Titannitrid auf ihrer dem Elektrodengrundkörper abgewandten Seite zumindest teilweise mit mindestens einer Oxidationsschutzschicht bedeckt ist. Dies ist insbesondere dann von Vorteil, wenn die Stimulationselektrode mit anodischer Polung eingesetzt werden soll, da hierdurch die Langzeitbeständigkeit der Stimulationselektrode deutlich erhöht wird.

Die Keramikschicht kann dabei auf der mindestens einen Oxidationsschutzschicht oder aber neben der elektrisch leitfähigen Beschichtung aus Titannitrid und der mindestens einen Oxidationsschutzschicht auf dem Elektrodengrundkörper angeordnet sein.

Weiterhin ist es möglich, dass die Keramikschicht neben der mindestens einen Oxidationsschutzschicht auf der Titannitridschicht angeordnet ist.

Für die mindestens eine Oxidationsschutzschicht hat es sich insbesondere bewährt, diese aus mindestens einem Element der Gruppe Platin, Iridium und Gold zu bilden. Es ist aber auch möglich, die Oxidationsschutzschicht aus einem Oxid, einem Karbid, einem Nitrid oder einem Polymer zu bilden, wobei die mindestens eine Oxidationsschutzschicht die Impedanz des mit der elektrisch leitfähigen Beschichtung aus Titannitrid beschichteten Elektrodengrundkörpers senkt oder auf maximal einen Wert erhöht, der kleiner ist als die Impedanz des unbeschichteten Elektrodengrundkörpers.

Vorteilhaft ist dabei eine Dicke der Oxidationsschutzschicht im Bereich von 500 nm bis 5 um.

Für die Keramikschicht haben sich Dicken im Bereich von 1 nm bis 20 µm bewährt, wobei die Schichtdicke stark vom Herstellungsverfahren abhängt, welches zur Bildung der Keramikschicht verwendet wird.

Die Keramikschicht kann dabei eine in sich geschlossene Fläche aufweisen oder aber aus mehreren nicht zusammenhängenden Flächen gebildet sein.

Die Aufgabe wird für ein erstes Verfahren zur Herstellung der Stimulationselektrode dadurch gelöst, dass die Keramikschicht durch ein PVD- (Physical Vapour Deposition) oder ein CVD- (Chemical Vapour Deposition) oder ein Tauch- oder ein Sprüh- oder ein Sol-Gel-Verfahren gebildet wird. Als geeignete PVD-Verfahren kommen dabei beispielsweise das Kathodenzerstäuben, thermisches Aufdampfen, Elektrodenstrahlverdampfen oder Lichtbogenverdampfen in Betracht.

Die Aufgabe wird für ein zweites Verfahren zur Herstellung der Stimulationselektrode dadurch gelöst, dass die Keramikschicht dadurch gebildet wird, dass eine Metallschicht aus Titan und/oder Niobium und/oder Tantal und/oder Zirkonium und/oder Aluminium und/oder Silizium abgeschieden wird und dass anschließend eine thermische oder elektrochemische oder chemische Oxidation und/oder Nitrierung der Metallschicht erfolgt. Dieses Verfahren ist in besonders einfacher und kostengünstiger Weise ausführbar.

Die Aufgabe wird für ein drittes Verfahren zur Herstellung einer Stimulationselektrode mit einem Elektrodengrundkörper aus Titan dadurch gelöst, dass die Keramikschicht gebildet wird, indem der Elektrodengrundkörper teilweise thermisch oder chemisch zu Titanoxid oxidiert wird.

Die Aufgabe wird für ein viertes Verfahren zur Herstellung einer Stimulationselektrode mit einem Elektrodengrundkörper aus Tantal dadurch gelöst, dass die Keramikschicht gebildet wird, indem der Elektrodengrundkörper teilweise thermisch oder elektrochemisch zu Tantaloxid oxidiert wird.

Das dritte und vierte erfindungsgemäße Verfahren ermöglicht die Bildung der Keramikschicht direkt aus dem Material des Elektrodengrundkörpers, so dass ein separater Materialauftrag für die Keramikschicht nicht erforderlich ist.

Die Aufgabe wird für ein fünftes Verfahren zur Herstellung einer Stimulationselektrode, bei welcher der Elektrodengrundkörper zumindest teilweise mit einer elektrisch leitfähigen Beschichtung aus Titannitrid bedeckt ist, dadurch gelöst, dass die Keramikschicht gebildet wird, indem die elektrisch leitfähige Beschichtung aus Titannitrid teilweise thermisch oder elektrochemisch zu Titanoxid oxidiert wird, wobei die Oxidation mindestens einen Teil der Schichtdicke der elektrisch leitfähigen Beschichtung aus Titannitrid umfasst.

Für das zweite bis fünfte erfindungsgemäße Verfahren hat es sich insbesondere bewährt, die thermische Oxidation mittels eines Lasers vorzunehmen.

Für das erste bis fünfte Verfahren hat es sich weiterhin bewährt, wenn die Keramikschicht zuerst vollflächig auf dem unbeschichteten oder beschichteten Elektrodengrundkörper gebildet wird und dass sie dann teilweise durch Ätzen wieder entfernt wird.

Die Verwendung der erfindungsgemäßen Stimulationselektrode als Herzschrittmacherelektrode, Neurostimulationselektrode, Retinaimplantat, Cochlearimplantat oder in einem anderen Human-Implantat ist ideal.

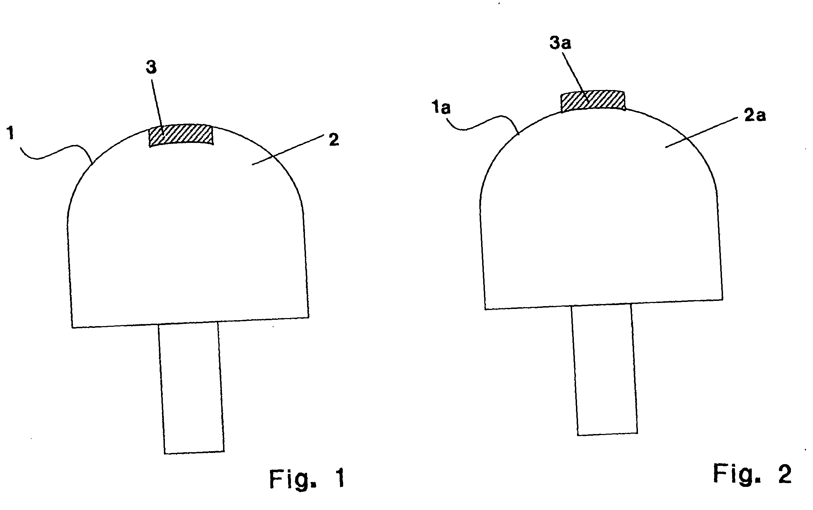

Zur Veranschaulichung der Erfindung zeigen die Figuren 1 bis 8 beispielhaft erfindungsgemäße Stimulationselektroden, wobei ausdrücklich hinzugefügt wird, dass die Stimulationselektrode auch jegliche andere Form, wie zum Beispiel eine ringförmige, wendelförmige oder flache Ausgestaltung, annehmen kann als die hier dargestellte.

So zeigen:
- Figur 1: einen Elektrodengrundkörper aus Titan mit einer Keramikschicht
- Figur 2: einen Elektrodengrundkörper aus Edelstahl mit einer Keramikschicht
- Figur 3: eine Stimulationselektrode mit einem Elektrodengrundkörper aus Titan mit einer Titannitridbeschichtung
- Figur 4: eine Stimulationselektrode mit einem Elektrodengrundkörper aus einer Platin-Iridium-Legierung
- Figuren 5 bis 8: Stimulationselektroden mit einem Elektrodengrundkörper aus Titan mit einer Titannitridbeschichtung, die eine Oxidationsschutzschicht aufweist

Figur 1 zeigt ein Zwischenprodukt 1 bei der Herstellung der erfindungsgemäßen Stimulationselektrode mit einem Elektrodengrundkörper 2 aus Titan, welcher oberflächig mittels eines Lasers zu einer Keramikschicht 3 aus Titanoxid oxidiert wurde.

Figur 2 zeigt ein Zwischenprodukt 1a bei der Herstellung der erfindungsgemäßen Stimulationselektrode mit einem Elektrodengrundkörper 2a aus Edelstahl. Auf dem Elektrodengrundkörper 2a ist eine Keramikschicht 3a aus Aluminiumoxid angeordnet, welche durch Elektronenstrahlverdampfen gebildet wurde.

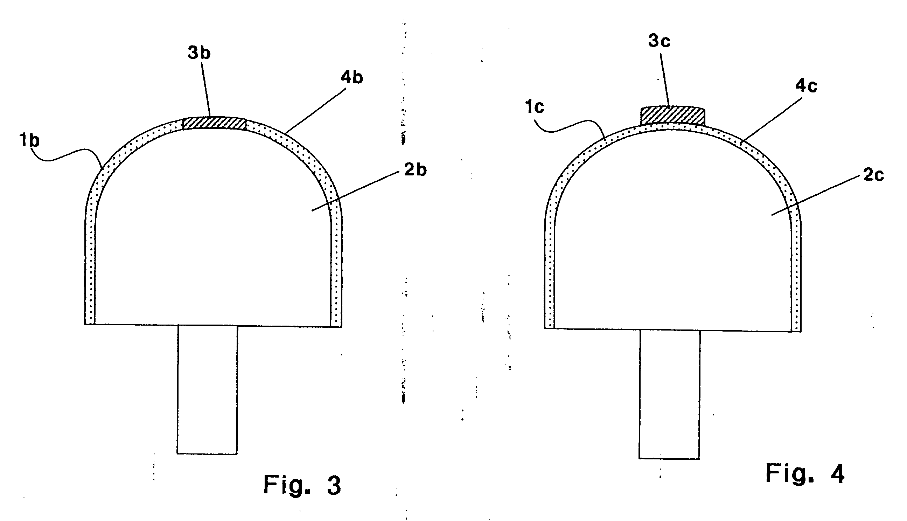

Figur 3 zeigt eine erfindungsgemäße Stimulationselektrode 1 b mit einem Elektrodengrundkörper 2b aus Titan. Der Elektrodengrundkörper 2b ist dabei mit einer Titannitridschicht 4b beschichtet. Die Titannitridschicht 4b wurde mittels elektrochemischer Oxidation teilweise unter Bildung der Keramikschicht 3b zu Titanoxid oxidiert.

Figur 4 zeigt eine erfindungsgemäße Stimulationselektrode 1 c mit einem Elektrodengrundkörper 2c aus einer Platin - Iridium Legierung mit 10 Gew.-% Iridiumanteil. Der Elektrodengrundkörper 2c ist dabei mit einer Schicht 4c aus Iridiumoxid bedeckt. Auf der Schicht 4c ist eine Keramikschicht 3c aus Tantaloxid angeordnet, welche durch Aufdampfen von metallischem Tantal und nachfolgender thermischer Oxidation des Tantals gebildet wurde.

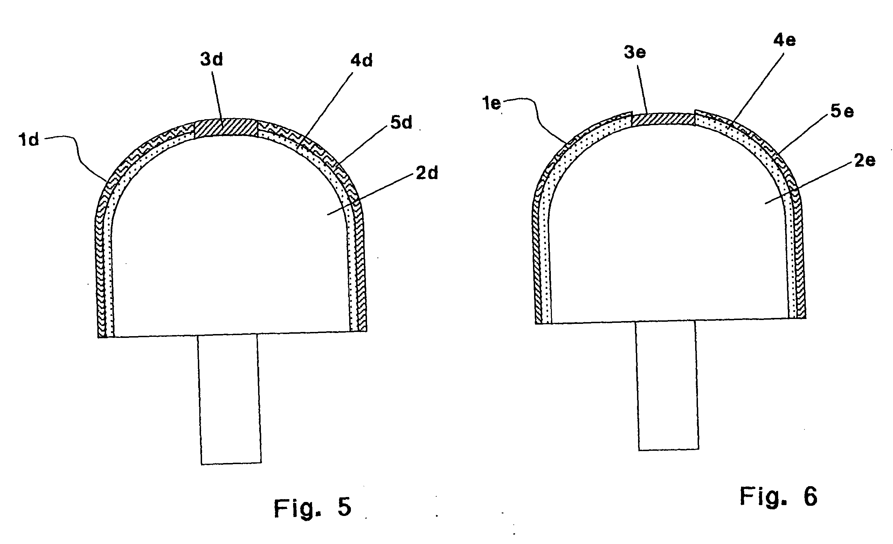

Figur 5 zeigt eine erfindungsgemäße Stimulationselektrode 1d mit einem Elektrodengrundkörper 2d aus Titan. Der Elektrodengrundkörper 2d ist mit einer Titannitridschicht 4d bedeckt, welche wiederum mit einer Oxidationsschutzschicht 5d aus Iridium überzogen ist. Der Titannitridschicht 4d sowie der Oxidationsschutzschicht 5d aus Iridium ist eine Keramikschicht 3d aus Titanoxid nebengeordnet, welche direkt auf dem Elektrodengrundkörper 2d angeordnet ist. Die Keramikschicht 3d ist dabei aus Titanoxid gebildet, welches durch Aufbringen von metallischem Titan und nachfolgender thermischer Oxidation mittels eines Lasers gebildet wurde.

Figur 6 zeigt eine erfindungsgemäße Stimulationselektrode 1e mit einem Elektrodengrundkörper 2e aus Titan. Der Elektrodengrundkörper 2e ist dabei mit einer Titannitridschicht 4e bedeckt, die wiederum teilweise mit einer Oxidationsschutzschicht 5e aus Platin bedeckt ist. In dem Bereich, in welchem die Oxidationsschutzschicht 5e die Titannitridschicht 4e nicht bedeckt, wird durch Oxidation der Titannitridschicht 4e eine Keramikschicht 3e aus Titanoxid gebildet.

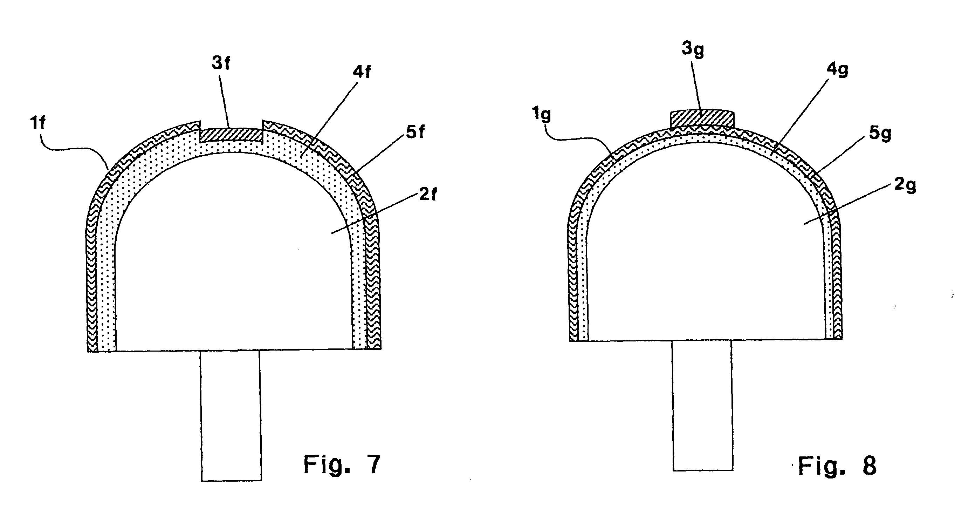

Figur 7 zeigt eine erfindungsgemäße Stimulationselektrode 1f mit einem Elektrodengrundkörper 2f aus Titan, welcher mit einer Titannitridschicht 4f beschichtet ist. Die Titannitridschicht 4f ist teilweise mit einer Oxidationsschutzschicht 5f aus Iridium beschichtet. In den nicht von der Oxidationsschutzschicht 5f bedeckten Bereichen der Titannitridschicht 4f ist die Titannitridschicht 4f oberflächlich in eine Keramikschicht 3f aus Titanoxid mittels thermischer Oxidation durch einen Laser umgewandelt.

Figur 8 zeigt eine Stimulationselektrode 1 g mit einem Elektrodengrundkörper 2g aus Titan, welcher mit einer Titannitridschicht 4g bedeckt ist. Die Titannitridschicht 4g ist wieder mit einer Oxidationsschutzschicht 5g aus einer Platin-Iridium-Legierung bedeckt. Auf der Oxidationsschutzschicht 5g ist eine Keramikschicht 3g aus Zirkoniumoxid angeordnet, welche durch ein CVD-Verfahren gebildet wurde.

## Patentansprüche

1. Stimulationselektrode mit einem elektrisch leitenden Elektrodengrundkörper, der teilweise mit einer elektrisch isolierenden Keramikschicht bedeckt ist, wobei die Keramikschicht ( 3, 3a, 3b, 3c, 3d, 3e, 3f, 3g ) aus einem Oxid und/oder einem Oxinitrid mindestens eines Metalls der Gruppe umfassend Titan, Niobium, Tantal, Zirkonium, Aluminium und Silizium gebildet ist, **dadurch gekennzeichnet, dass** der Elektrodengrundkörper ( 2, 2a, 2b, 2c, 2d, 2e, 2f, 2g ) weiterhin zumindest teilweise mit einer elektrisch leitfähigen Beschichtung ( 4a, 4b, 4c, 4d, 4e, 4f, 4g ) aus Titannitrid, Niobiumnitrid, Tantalnitrid, Zirkoniumnitrid, Aluminiumnitrid, Siliziumnitrid, Vanadiumnitrid, Iridiumoxid oder einer Legierung aus Platin und Iridium beschichtet ist, wobei der Iridium-Anteil der Legierung ≥ 21 Gew.-% beträgt und der Platin-Anteil der Legierung ≥ 100 ppm beträgt.

2. Stimulationselektrode nach Anspruch 1, **dadurch gekennzeichnet, dass** der Elektrodengrundkörper ( 2, 2a, 2b, 2c, 2d, 2e, 2f, 2g ) aus Titan, Tantal, Gold, Kohlenstoff, Platin, Iridium, einer Platin-Iridium-Legierung, einer auf Kobalt und/oder Nickel basierenden Legierung oder Edelstahl gebildet ist.

3. Stimulationselektrode nach einem der Ansprüche 1 bis 2, **dadurch gekennzeichnet, dass** die Keramikschicht ( 3, 3a, 3b, 3c, 3d, 3e, 3f, 3g ) auf der elektrisch leitfähigen Beschichtung ( 4c ) angeordnet ist.

4. Stimulationselektrode nach einem der Ansprüche 1 bis 2, **dadurch gekennzeichnet, dass** die Keramikschicht ( 3, 3a, 3b, 3c, 3d, 3e, 3f, 3g ) neben der elektrisch leitfähigen Beschichtung (4a) auf dem Elektrodengrundkörper ( 2, 2a, 2b, 2c, 2d, 2e, 2f, 2g ) angeordnet ist.

5. Stimulationselektrode nach einem der Ansprüche 1 bis 4, **dadurch gekennzeichnet, dass** die elektrisch leitfähige Beschichtung ( 4b, 4d, 4e, 4f, 4g ) aus Titannitrid gebildet ist.

6. Stimulationselektrode nach Anspruch 5, **dadurch gekennzeichnet, dass** die elektrisch leitfähige Beschichtung ( 4b, 4d, 4e, 4f, 4g ) aus Titannitrid auf ihrer dem Elektrodengrundkörper ( 2, 2a, 2b, 2c, 2d, 2e, 2f, 2g ) abgewandten Seite zumindest teilweise mit mindestens einer Oxidationsschutzschicht ( 5d, 5e, 5f, 5g ) bedeckt ist.

7. Stimulationselektrode nach Anspruch 6, **dadurch gekennzeichnet, dass** die Keramikschicht ( 3, 3a, 3b, 3c, 3d, 3e, 3f, 3g ) auf der mindestens einen Oxidationsschutzschicht ( 5d, 5e, 5f, 5g ) angeordnet ist.

8. Stimulationselektrode nach Anspruch 6, **dadurch gekennzeichnet, dass** die Keramikschicht ( 3, 3a, 3b, 3c, 3d, 3e, 3f, 3g ) neben der elektrisch leitfähigen Beschichtung (4b, 4d, 4e, 4f, 4g ) aus Titannitrid und der mindestens einen Oxidationsschutzschicht ( 5d, 5e, 5f, 5g ) auf dem Elektrodengrundkörper ( 2, 2a, 2b, 2c, 2d, 2e, 2f, 2g ) angeordnet ist.

9. Stimulationselektrode nach Anspruch 6, **dadurch gekennzeichnet, dass** die Keramikschicht ( 3, 3a, 3b, 3c, 3d, 3e, 3f, 3g ) neben der mindestens einen Oxidationsschutzschicht ( 5d, 5e, 5f, 5g ) auf der elektrisch leitfähigen Beschichtung ( 4b, 4d, 4e, 4f, 4g ) aus Titannitrid angeordnet ist.

10. Stimulationselektrode nach einem der Ansprüche 6 bis 9, **dadurch gekennzeichnet, dass** die Oxidationsschutzschicht ( 5d, 5e, 5f, 5g ) aus mindestens einem Element der Gruppe Platin, Iridium und Gold gebildet ist.

11. Stimulationselektrode nach einem der Ansprüche 6 bis 9, **dadurch gekennzeichnet, dass** die Oxidationsschutzschicht ( 5d, 5e, 5f, 5g ) aus einem Oxid, einem Karbid, einem Nitrid oder einem Polymer gebildet ist, wobei die mindestens eine Oxidationsschutzschicht ( 5d, 5e, 5f, 5g ) die Impedanz des mit der elektrisch leitfähigen Beschichtung ( 4b, 4d, 4e, 4f, 4g ) aus Titannitrid beschichteten Elektrodengrundkörpers ( 2, 2a, 2b, 2c, 2d, 2e, 2f, 2g ) senkt oder auf maximal einen Wert erhöht, der kleiner ist als die Impedanz des unbeschichteten Elektrodengrundkörpers ( 2, 2a, 2b, 2c, 2d, 2e, 2f, 2g ).

12. Stimulationselektrode nach einem der Ansprüche 6 bis 11, **dadurch gekennzeichnet, dass** die Oxidationsschutzschicht ( 5d, 5e, 5f, 5g ) eine Dicke im Bereich von 500nm bis 5µm aufweist.

13. Stimulationselektrode nach einem der Ansprüche 1 bis 12, **dadurch gekennzeichnet, dass** die Keramikschicht ( 3, 3a, 3b, 3c, 3d, 3e, 3f, 3g ) eine Dicke im Bereich von 1 nm bis 20µm aufweist.

14. Stimulationselektrode nach einem der Ansprüche 1 bis 13, **dadurch gekennzeichnet, dass** die Keramikschicht ( 3, 3a, 3b, 3c, 3d, 3e, 3f, 3g ) eine in sich geschlossene Fläche aufweist.

15. Stimulationselektrode nach einem der Ansprüche 1 bis 13, **dadurch gekennzeichnet, dass** die Keramikschicht ( 3, 3a, 3b, 3c, 3d, 3e, 3f, 3g ) mehrere nicht zusammenhängende Flächen aufweist.

16. Verfahren zur Herstellung einer Stimulationselektrode nach einem der Ansprüche 1 bis 15, **dadurch gekennzeichnet, dass** die Keramikschicht ( 3, 3a, 3b, 3c, 3d, 3e, 3f, 3g ) durch ein PVD- oder ein CVD- oder ein Tauch- oder ein Sprüh- oder ein Sol-Gel-Verfahren gebildet wird.

17. Verfahren zur Herstellung einer Stimulationselektrode nach einem der Ansprüche 1 bis 15, **dadurch gekennzeichnet, dass** die Keramikschicht ( 3, 3a, 3b, 3c, 3d, 3e, 3f, 3g ) dadurch gebildet wird, dass eine Metallschicht aus Titan und/oder Niobium und/oder Tantal und/oder Zirkonium und/oder Aluminium und/oder Silizium abgeschieden wird und dass anschließend eine thermische oder elektrochemische oder chemische Oxidation oder Oxinitrierung der Metallschicht erfolgt.

18. Verfahren zur Herstellung einer Stimulationselektrode nach einem der Ansprüche 1 bis 2 oder 4, **dadurch gekennzeichnet, dass** die Keramikschicht ( 3, 3a, 3b, 3c, 3d, 3e, 3f, 3g ) dadurch gebildet wird, dass ein Elektrodengrundkörper ( 2, 2a, 2b, 2c, 2d, 2e, 2f, 2g ) aus Titan teilweise thermisch oder elektrochemisch zu Titanoxid oxidiert wird.

19. Verfahren zur Herstellung einer Stimulationselektrode nach einem der Ansprüche 1 bis 2 oder 4, **dadurch gekennzeichnet, dass** die Keramikschicht ( 3, 3a, 3b, 3c, 3d, 3e, 3f, 3g ) dadurch gebildet wird, dass ein Elektrodengrundkörper ( 2, 2a, 2b, 2c, 2d, 2e, 2f, 2g ) aus Tantal teilweise thermisch oder elektrochemisch zu Tantaloxid oxidiert wird.

20. Verfahren zur Herstellung einer Stimulationselektrode nach einem der Ansprüche 3 und 5 oder 5 und 9, **dadurch gekennzeichnet, dass** die Keramikschicht ( 3, 3a, 3b, 3c, 3d, 3e, 3f, 3g ) dadurch gebildet wird, dass die elektrisch leitfähige Beschichtung ( 4b, 4d, 4e, 4f, 4g ) aus Titannitrid teilweise thermisch oder elektrochemisch zu Titanoxid oxidiert wird, wobei die Oxidation mindestens einen Teil der Schichtdicke der elektrisch leitfähigen Beschichtung ( 4b, 4d, 4e, 4f, 4g ) aus Titannitrid umfasst.

21. Verfahren nach Anspruch 17, 18, 19 oder 20, **dadurch gekennzeichnet, dass** die thermische Oxidation mittels eines Lasers erfolgt.

22. Verfahren nach einem der Ansprüche 16 bis 21, **dadurch gekennzeichnet, dass** die Keramikschicht ( 3, 3a, 3b, 3c, 3d, 3e, 3f, 3g ) zuerst vollflächig auf dem unbeschichteten oder beschichteten Elektrodengrundkörper ( 2, 2a, 2b, 2c, 2d, 2e, 2f, 2g ) gebildet wird und dass sie dann teilweise durch Ätzen wieder entfernt wird.

23. Verwendung einer Stimulationselektrode nach einem der Ansprüche 1 bis 15 als Herzschrittmacherelektrode, Neurostimulationselektrode oder in einem anderen Human-Implantat.
